# EUROPEAN PATENT APPLICATION

(11) **EP 1 508 605 A1**
(43) Date of publication of application: **23.02.2005**
(21) Application number: 03755285.8
(22) Date of filing: 26.05.2003
(51) Int. Cl.: C09K 3/00, A61K 7/42

(54) **METHOD FOR PRODUCING PURIFIED ULTRAVIOLET RAY ABSORBING AGENT**

(30) Priority: 27.05.2002 JP 2002152932
(71) Applicant: The Nisshin OilliO Group, Ltd., Tokyo 104-8285 (JP)
(72) Inventor: Iwamoto, Y. The Nisshin OilliO, Ltd., Yokohama-shi, Kanagawa 235-8558 (JP); Saida, T. The Nisshin OilliO, Ltd., Yokohama-shi, Kanagawa 235-8558 (JP); Nakamura, Kayo The Nisshin OilliO, Ltd., Yokohama-shi, Kanagawa 235-8558 (JP)
(74) Representative: Vuillermoz, Bruno
(86) International application number: PCT/JP2003/006547
(87) International publication number: WO 2003/099956

(57) **Abstract**

An object of the present invention is to provide a production method for a purified ultraviolet absorbing agent having less odor, excellent safety, as well as improved hue, which has improved industrial productivity (production cost), fewer production processes, improved safety during production, and less effect on the environment. In order to achieve this object, the present invention provides a production method for a purified ultraviolet absorbing agent comprising an absorbent treatment process in which an ultraviolet absorbing agent is contacted directly with an absorbent without using a solvent.

## Description

### TECHNICAL FIELD

The present invention relates to a production method for a purified ultraviolet absorbing agent and relates to a purified ultraviolet absorbing agent produced by the production method. More specifically, the present invention relates to a production method for a purified ultraviolet absorbing agent having reduced odor, excellent safety, and improved color, with excellent industrial productivity, and relates to a purified ultraviolet absorbing agent produced by the production method.

### BACKGROUND ART

An ultraviolet absorbing agent has been used to obtain a cosmetic composition having an effect of protecting against ultraviolet light. Conventional ultraviolet absorbing agents include well-known synthetic esters such as ethyl paramethoxy cinnamate, isopropyl paramethoxy cinnamate, 2-ethylhexyl paramethoxy cinnamate, glyceryl 2-ethylhexanoate di-p-methoxy cinnamate, octyl salicylate, phenyl salicylate, homomenthyl salicylate, dipropylene glycol salicylate, ethylene glycol salicylate, myristyl salicylate, and methyl salicylate.

In general, an antioxidant such as butylhydroxytoluene (BHT) or a tocopherol is used together with these ultraviolet absorbing agents. However, even when an antioxidant is used, since almost all of the ultraviolet absorbing agents are compounds having an aromatic structure or derivatives thereof, they have peculiar odors. Because of the peculiar odors, there are cases in which a sufficient amount of an ultraviolet absorbing agent cannot be added to a cosmetic composition to be effective.

Japanese Unexamined Patent Application, First Publication No. H7-89835 discloses, as a means for improving the odor of ultraviolet absorbing agents, a method in which an ultraviolet absorbing agent is diluted in a solvent such as hexane, and then is purified using an adsorbent. According to this method, butylhydroxytoluene or a tocopherol, which is added as an antioxidant, is adsorbed together with an odor component, and a sufficient amount of the antioxidant to prevent oxidation does not remain. Due to this, this method has a problem in that suppression of discoloring effects is not obtained over time.

In addition, a large amount of a solvent or an adsorbent, which is used in industrial production, causes environmental problems, and this is not desirable in practical use.

An object of the present invention is to provide a production method for a purified ultraviolet absorbing agent which has reduced odor and which is excellent in safety and is improved in color, with excellent industrial productivity.

Another object of the present invention is to provide a purified ultraviolet absorbing agent produced by the production method, which has reduced odor and which is excellent in safety and is improved in color.

### DISCLOSURE OF THE INVENTION

In order to achieve the objects, as a result of conducting diligent research that focused on purification of synthetic esters, which are mainly used as ultraviolet absorbing agents, the inventors found that a production method for a purified ultraviolet absorbing agent comprising an adsorbent treatment step in which a raw material of a ultraviolet adsorbing agent is directly contacted with an adsorbent without using a solvent, can achieve the objects.

In other words, in order to achieve the objects, the present invention provides a production method for a purified ultraviolet adsorbing agent comprising an adsorbent treatment step in which a raw material of a ultraviolet adsorbing agent is directly contacted with an adsorbent without using a solvent. The production method of the present invention has improved industrial productivity (production cost), fewer production processes, improved safety during production, and does not have adverse effects on the environment.

The ultraviolet adsorbing agent used in the present invention is preferably an ester compound which comprises at least one fatty acid having an aromatic structure and monovalent or polyvalent alcohol. Among these esters, an ester compound comprising at least methoxy cinnamate and monovalent or polyvalent alcohol, and 2-ethylhexyl paramethoxy cinnamate is more preferable.

In the production method, it is preferable for the adsorbent used to be non-hydrated activated alumina, non-hydrated activated magnesia, a complex of non-hydrated activated alumina and non-hydrated activated magnesia, or a mixture thereof.

In the production method, it is preferable to have a deodorization treatment step for removing odor of the ultraviolet adsorbing agent after the adsorbent treatment step.

In addition, in order to achieve the objects, the present invention provides a purified ultraviolet absorbing agent which is produced by the production method.

### BEST MODE FOR CARRYING OUT THE INVENTION

As explained above, in the production method of the present invention, the ultraviolet absorbing agent is an ester compound which comprises mainly at least one fatty acid having an aromatic structure and monovalent or polyvalent alcohol. For example, the ultraviolet absorbing agent includes synthetic esters such as ethyl paramethoxy cinnamate, isopropyl paramethoxy cinnamate, 2-ethylhexyl paramethoxy cinnamate, mono-2-ethylhexanoic glyceryl diparamethoxycinnamate, octyl salicylate, phenyl salicylate, homomenthyl salicylate, dipropylene glycol salicylate, ethylene glycol salicylate, miristyl salicylate, and methyl salicylate. These synthetic esters may be used individually or in combination of two or more thereof. In addition, these synthetic esters can be used together with the other synthetic esters which are widely used. Among these, an ester compound produced by at least methoxy cinnamate and monovalent or polyvalent alcohol, or a mixture containing the ester compound and well-known synthetic ester is more preferable.

A synthetic adsorbent containing oxides (or hydroxides) of Mg, Al, and Si is preferable as the adsorbent used in the present invention. Among these, an activated alumina, an activated magnesia, and a complex thereof are more preferable. In addition, among these, adsorbents which are not hydrated and are dried are most preferable. These adsorbents may be used individually or in combination of two or more thereof.

During the adsorbent treatment step, the adsorbent is directly added to the ultraviolet adsorbing agent, and then this is stirred at a normal temperature and under normal pressure, and after that, the adsorbent is removed by filtering.

The amount of adsorbent used is preferably in a range from 1 to5 % by mass relative to 100% by mass of a raw material of the ultraviolet adsorbing agent. Even if it exceeds 5% by mass, the adsorbent treatment can be conducted. However, since 5% by mass or less of the adsorbent provides desired effects, from the viewpoint of industrial productivity and effects on the environment, the amount of adsorbent used is preferably in a range from 1 to 5% by mass relative to 100% by mass of a raw material of the ultraviolet adsorbing agent.

As the deodorization treatment step after the adsorbent treatment step of the production method according to the present invention, an ordinary deodorization treatment method for synthetic esters, raw materials of oils and fats, and tocopherols may be conducted. As explained above, the deodorization treatment in the present invention is preferably conducted after the adsorbent treatment. When the deodorization treatment is conducted before the adsorbent treatment, strong odors may be generated together with worse of hue, and a purification using the above amount of the adsorbent is insufficient. Furthermore, when the mixture containing the raw material of the ultraviolet adsorbing agent and the adsorbent is heated, peculiar odors are generated and sufficient purification is impossible by only filtering the adsorbent.

In particular, when heating under reduced pressure is conducted during the deodorization treatment step, both nitrogen gas and water vapor can be used as blown gas. The degree of vacuum in heating under reduced pressure is preferably 4,000 Pa or less, and more preferably 1,000 Pa or less. The heating temperature is preferably in a range from 30 to 250°C, and more preferably in a range from 80 to 200°C.

Below, the present invention will be explained in detail with reference to the following Examples. The present invention is not limited to the following Examples.

### Example 1

2-ethylhexyl paramethoxy cinnamate was used as the ultraviolet adsorbing agent to be purified. In a vessel provided with an agitator, 1,000 g of 2-ethylhexyl paramethoxy cinnamate, which is not purified yet, and 10 g of an activated alumina·magnesia anhydride as the adsorbent were placed, the mixture was stirred for 1 hour, and then the mixture was filtered, and thereby a purified ultraviolet adsorbing agent was obtained.

After that, the obtained purified ultraviolet adsorbing agent was placed into a three-necked flask provided with a nitrogen gas blow pipe and a thermometer. While blowing nitrogen gas, it was heated under reduced pressure for 3 hours at 100 to 140°C, and thereby the deodorization treatment was performed. Then, a purified ultraviolet adsorbing agent in this Example was obtained.

After that, odor and hue of the purified ultraviolet adsorbing agent in this Example were evaluated as shown below. In addition, 100 ml of the obtained purified ultraviolet adsorbing agent was put into a 200 ml sample bottle, and a sample was prepared. The prepared sample was allowed to stand for 3 weeks in the dark (temperature: 50°C). Another sample was allowed to stand for 3 weeks under illumination (temperature: 20°C; illumination: 1,000 lux). After being left for 3 weeks, the odor of each sample was evaluated in a similar manner.

### Evaluation of odor

Strength of total odor and irritating odor was categorized from 1 (non-odor) to 10 (extremely strong odor) ranks.

### Evaluation of hue

Using a Nessleriser 2150 (marketed by Lovibond), hue was evaluated in accordance with Hazen color (APHA).

### Example 2

A purified ultraviolet adsorbing agent was prepared and evaluated in a manner identical to that of Example 1, except that nitrogen used in the deodorization treatment step was replaced with water vapor.

### Example 3

A purified ultraviolet adsorbing agent was prepared and evaluated in a manner identical to that of Example 1, except that 50 g of an activated alumina·magnesia anhydride was used as the adsorbent.

### Example 4

A purified ultraviolet adsorbing agent was prepared and evaluated in a manner identical to that of Example 1, except that 100 g of an activated alumina·magnesia anhydride was used as the adsorbent.

### Example 5

A purified ultraviolet adsorbing agent was prepared and evaluated in a manner identical to that of Example 1, except that an activated alumina was used as the adsorbent.

### Example 6

A purified ultraviolet adsorbing agent was prepared and evaluated in a manner identical to that of Example 1, except that an activated magnesia was used as the adsorbent.

### Example 7

A purified ultraviolet adsorbing agent was prepared and evaluated in a manner identical to that of Example 1, except that 5 g of an activated alumina·magnesia anhydride was used as the adsorbent.

### Example 8

A purified ultraviolet adsorbing agent was prepared and evaluated in a manner identical to that of Example 1, except that the deodorization treatment was not conducted.

### Example 9

A purified ultraviolet adsorbing agent was prepared and evaluated in a manner identical to that of Example 1, except that the adsorbent treatment was conducted after the deodorization treatment.

### Example 10

A purified ultraviolet adsorbing agent was prepared and evaluated in a manner identical to that of Example 1, except that an activated clay was used as the adsorbent.

### Example 11

A purified ultraviolet adsorbing agent was prepared and evaluated in a manner identical to that of Example 1, except that silica gel was used as the adsorbent.

### Example 12

A purified ultraviolet adsorbing agent was prepared and evaluated in a manner identical to that of Example 1, except that an activated alumina·magnesia hydrate was used as the adsorbent.

### Example 13

A purified ultraviolet adsorbing agent was prepared and evaluated in a manner identical to that of Example 1, except that an activated silica alumina hydrate was used as the adsorbent.

### Example 14

A purified ultraviolet adsorbing agent was prepared and evaluated in a manner identical to that of Example 1, except that the filtration of the adsorbent was conducted after heating treatment under reduced pressure rather than before.

### Comparative Example 1

Evaluation was performed using 2-ethylhexyl paramethoxy cinnamate, which was used as the ultraviolet adsorbing agent to be purified without the adsorbent treatment and the deodorization treatment.

### Comparative Example 2

A purified ultraviolet adsorbing agent was prepared and evaluated in a manner identical to that of Example 1, except that only the deodorization treatment was conducted, and the adsorbent treatment was not conducted

### Comparative Example 3

A purified ultraviolet adsorbing agent was prepared and evaluated in a manner identical to that of Example 1, except that 1,000 g of hexane was added as a solvent relative to 1,000 g of 2-ethylhexyl paramethoxy cinnamate, which was a ultraviolet adsorbing agent to be purified.

The evaluation results are shown in the following Table 1.

**Table 1**

| | Evaluation after purification or non-purification | | Evaluation after being allowed to stand | |
|---|---|---|---|---|
| | Odor | Hue | Odor after standing in the dark | Order after standing under illumination |
| Example 1 | 1 | 10 | 2 | 1 |
| Example 2 | 1 | 10 | 2 | 1 |
| Example 3 | 1 | 10 | 2 | 1 |
| Example 4 | 1 | 10 | 2 | 1 |
| Example 5 | 1 | 20 | 2 | 1 |
| Example 6 | 1 | 20 | 2 | 1 |
| Example 7 | 2 | 30 | 3 | 2 |
| Example 8 | 9 | 10 | 5 | 4 |
| Example 9 | 6 | 10 | 4 | 3 |
| Example 10 | 4 | 20 | 3 | 3 |
| Example 11 | 3 | 20 | 3 | 2 |
| Example 12 | 3 | 20 | 3 | 2 |
| Example 13 | 3 | 20 | 3 | 2 |
| Example 14 | 2 | 10 | 3 | 2 |
| Comp. Example 1 | 10 | 40 | 5 | 4 |
| Comp. Example 2 | 7 | 40 | 4 | 4 |
| Comp. Example 3 | 1 | 10 | 2 | 1 |

The purified ultraviolet adsorbing agents prepared in the Examples have less odor and less changes in odor after standing than the ultraviolet adsorbing agents prepared in the Comparative Examples 1 and 2.

In addition, the purified ultraviolet adsorbing agent prepared in the Comparative Example 3 has an odor and changes in odor after standing which are of the same levels as the purified ultraviolet adsorbing agent in Example 1. However, when industrial productivity (production cost), effects on the environment, fewer production steps, and safety during production are considered, the production method in the Examples is superior to the production method in the Comparative Example 3.

### INDUSTRIAL APPLICABILITY

According to the production method for a purified ultraviolet adsorbing agent of the present invention, a production method, which produces a purified ultraviolet adsorbing agent having less odor, improved safety, and hue, at low cost and improved industrial productivity, can be produced.

## Claims

1. A production method for a purified ultraviolet adsorbing agent comprising:
an adsorbent treatment step in which a raw material of a ultraviolet adsorbing agent is directly contacted with an adsorbent without using a solvent.

2. A production method for a purified ultraviolet adsorbing agent according to claim 1, wherein said ultraviolet adsorbing agent is an ester compound which is produced by using at least one fatty acid having an aromatic structure and monovalent or polyvalent alcohol.

3. A production method for a purified ultraviolet adsorbing agent according to claim 1, wherein said ultraviolet adsorbing agent is an ester compound which is produced by using at least methoxy cinnamate and monovalent or polyvalent alcohol.

4. A production method for a purified ultraviolet adsorbing agent according to claim 1, wherein said ultraviolet adsorbing agent is 2-ethylhexyl paramethoxy cinnamate.

5. A production method for a purified ultraviolet adsorbing agent according to claim 1, wherein said adsorbent is one adsorbent selected from a non-hydrated activated alumina, a non-hydrated activated magnesia, a complex of a non-hydrated activated alumina and a non-hydrated activated magnesia, and a mixture thereof.

6. A production method for a purified ultraviolet adsorbing agent according to claim 1, wherein the production method further comprises a deodorization treatment step for removing odor of the ultraviolet adsorbing agent after the adsorbent treatment step.

7. A purified ultraviolet adsorbing agent which is produced by the production method of claim 1.
